(19) Europäisches Patentamt
European Patent Office
Office européen des brevets

(11) **EP 4 132 345 B1**

(12) **EUROPEAN PATENT SPECIFICATION**

(45) Date of publication and mention
of the grant of the patent:
**28.02.2024   Bulletin 2024/09**

(21) Application number: **21722546.5**

(22) Date of filing: **02.04.2021**

(51) International Patent Classification (IPC):
***A61B 3/16*** *(2006.01)*

(52) Cooperative Patent Classification (CPC):
**A61B 3/16**

(86) International application number:
**PCT/IB2021/052780**

(87) International publication number:
**WO 2021/205310 (14.10.2021 Gazette 2021/41)**

(54) **APPLANATION TONOMETER HEAD**

APPLANATIONSTONOMETERKOPF

TÊTE DE TONOMÈTRE À APLANATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **06.04.2020   IT 202000007261**

(43) Date of publication of application:
**15.02.2023   Bulletin 2023/07**

(73) Proprietor: **Frastema Ophthalmics Easyopht
Group S.r.l.
(in forma abbreviata FE - Group S.r.l.)
21058 Solbiate Olona VA (IT)**

(72) Inventor: **CARUSO, Ciro
80124 Napoli NA (IT)**

(74) Representative: **Barbaro, Gaetano et al
Società Italiana Brevetti S.p.A.
Via G. Carducci, 8
20123 Milano (IT)**

(56) References cited:
**US-A- 6 083 161     US-A1- 2001 051 770**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present disclosure generally relates to applanation tonometers and more particularly to an applanation tonometer head made of transparent material.

It is known that the measurement of intraocular pressure (IOP) represents a fundamental moment of routine ophthalmological exams, as the increase of its values represents the major risk factor for glaucoma and it is one of the fundamental parameters for the diagnosis of the disease. In addition, the IOP measurement provides useful indications for other eye diseases, such as uveitis, and in all patients undergoing eye surgery, so much so that it is considered a routine exam to be performed in all ophthalmological visits.

The measurement of intraocular pressure can be performed with direct (manometry) or indirect (tonometry) methods. Ocular manometry is not used in clinical practice as it is more invasive, but is limited only to experimental use. Instead, ocular tonometry is the most commonly used method in clinical practice. It allows the indirect measurement of intraocular pressure using instruments called tonometers, whose principle is based on the relationship between intraocular pressure and the force necessary to modify the natural shape of the cornea.

In the following, reference will only be made to the "applanation" tonometers, which measure the force necessary to flatten a known surface area of the cornea or evaluate the width of the area flattened by a predetermined fixed force, as it is to them that the invention applies.

The term "applanation tonometer", in the following description and in the claims, is intended to indicate any tonometer that uses the principle of applanation tonometry to measure intraocular pressure.

Applanation tonometry is based on the fact that, to flatten a surface of area A of the cornea, an average force F is required which acts on a surface of area A in order to balance the intraocular pressure (IOP):

$$\text{IOP (IntraOcular Pressure)} = F / A$$

It follows that the pressure inside an ideal sphere can be measured by evaluating the width of the corneal area flattened by a constant force or by evaluating the force required to flatten a known corneal area. Therefore, two different types of tonometers can be used for applanation tonometry: variable area and variable force.

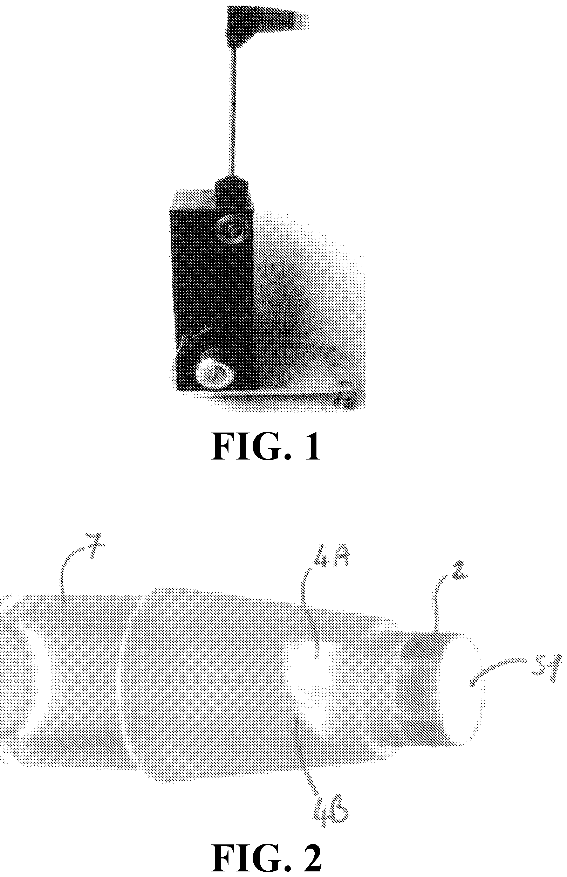

The most known and used applanation tonometers in the world are those with variable force and undoubtedly among these the most famous is the Goldmann tonometer, now universally used and which represents the international standard for measuring IOP. The Goldmann tonometer, represented in Figure 1, includes a flattening element, consisting of a head typically made of transparent plastic or glass and containing a prism, joined by means of a holding ring on a rod to a spiral spring. By means of a lateral graduated knob it is possible to vary the value of the force applied to the cornea, which is expressed in grams and is indicated by notches engraved on the knob.

The value of the flattened central cornea area is 3.06 mm$^2$ and this value, always constant, is set as a standard reference value.

The exam technique involves resting the instrument on a special base of a slit lamp. After corneal anesthesia, the folded end of a strip of filter paper soaked in fluorescein, a fluorescent substance that serves to make the edge of the flattened area more evident by observation with blue light, is applied in the lower conjunctival fornix of the patient. As is known, identifying the edges of the flattened area with good precision is essential for a correct measurement of ocular pressure.

Figure 2 is a perspective view of a classic applanation tonometer head. It appears externally as an object with substantially rotational symmetry along a longitudinal axial direction and has an integral body defining:

at a first end along the longitudinal axial direction, an end portion 2 defining a flat corneal applanation surface S1, having a respective applanation radius, arranged on a first base plane of the end portion 2 orthogonal to a longitudinal axis of the head for tonometer;

two semi-cylindrical prisms 4a and 4b integral with the end portion 2, each having:

- a respective lower semicircular base arranged along a respective plane orthogonal to the longitudinal axial direction;
- a nominal height at a center of the respective lower semicircular base;
- a semi-elliptical upper base surface opposite the respective lower base and inclined with respect thereto by a respective internal angle, typically comprised between 10 and 60°.

The two semi-cylindrical prisms 4a and 4b are opposite each other in the sense of height along an axial separation plane so that the respective lower semicircular bases lie on the same orthogonal plane composing together a full circle.

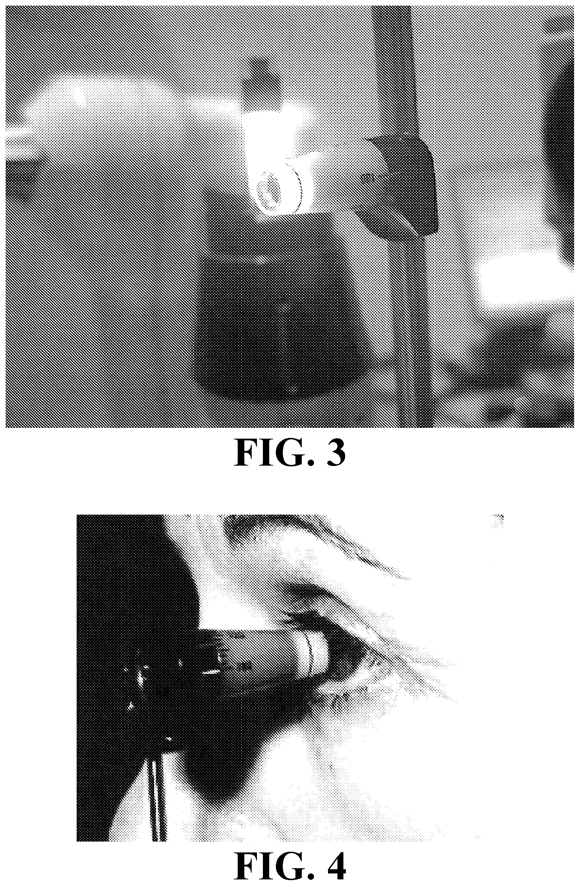

The known head of figure 2 typically has a tubular shank 7 which surrounds the upper base surfaces of the two prisms 4a and 4b, shown in semi-transparency, and is configured to be engaged in a holding ring of an applanation tonometer while maintaining the tonometer head outside the holding ring, as shown in figure 3. The lateral surface of the current tonometer heads is opaque, so as to shield the prisms 4a and 4b from spurious light rays coming from the side and which could disturb the vision, and notches are made on it to correctly align the plane of separation of the prisms 4a and 4b to the patient.

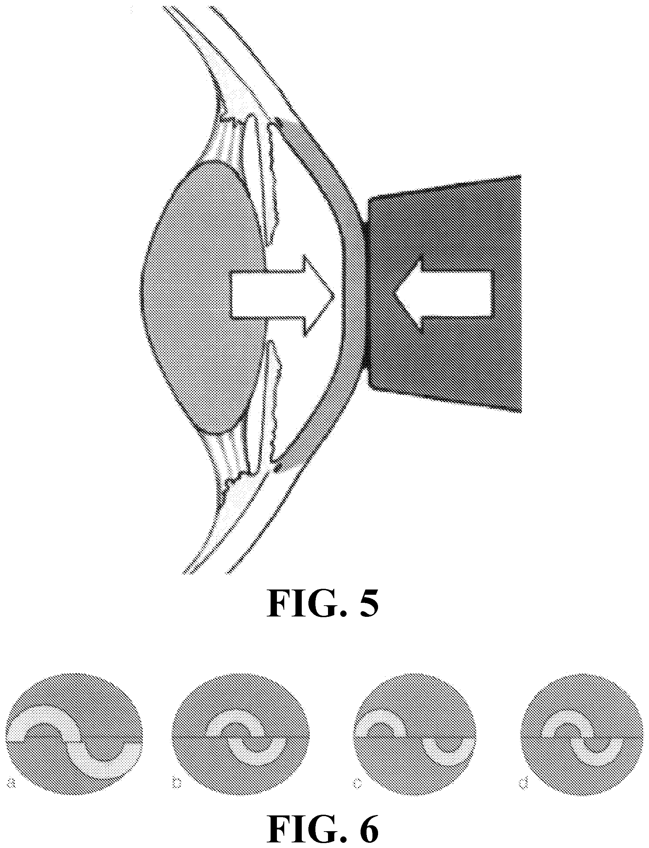

The patient is made to sit in front of the slit lamp and look at a reference point, as shown in figure 4. The slit of the lamp is opened to the maximum and a cobalt blue filter is interposed to better visualize the fluorescein, that is to better visualize the edges of the flattened area. A light beam is then directed so that it passes through the transparent prism and the knob is adjusted to the notch corresponding to 10 mmHg. The slit lamp is moved forward so that the prism gently contacts the central part of the cornea, as in figure 5.

The prism, contained in the transparent plastic head, splits the circular image of the flattened corneal surface into two semicircles, one on top of the other. Then through the eyepiece, it is possible to see two green semicircles in a blue luminous field.

Figures 6a-6d show what is observed with the Goldmann tonometer in different cases. The side knob of the tonometer is rotated so as to bring the two semicircles into contact with their inner edge. Depending on the exerted pressure, different aspects of the semicircles can be observed: if these are far away from each other, it is necessary to increase the applanation force; if they overlap it is necessary to rotate the knob in reverse to decrease the exerted force. In Figure 6a, the hemicorones appear relatively thick due to excessive use of fluorescein; in figure 6b the hemicorones have an adequate thickness but are not correctly aligned because the applanation force is excessive; in figure 6c instead the applanation force is insufficient; in figure 6d the hemicorones are correctly aligned. At this point the IOP is determined by multiplying the value shown on the knob by ten.

Document US 6 083 161 A, which is considered to represent the most relevat prior art, discloses an applanation tonometer transducer body with a corneal contact surface. The tonometer/pachymeter of D1 also includes an opposing prism assembly which has an upper prism and a lower prism separated by a prism delineation line. Document US 2001/051770 A1 discloses a prism having a longitudinal axis centered on a preferably circular applanation surface and providing a viewing axis for a tonometer operator. 5

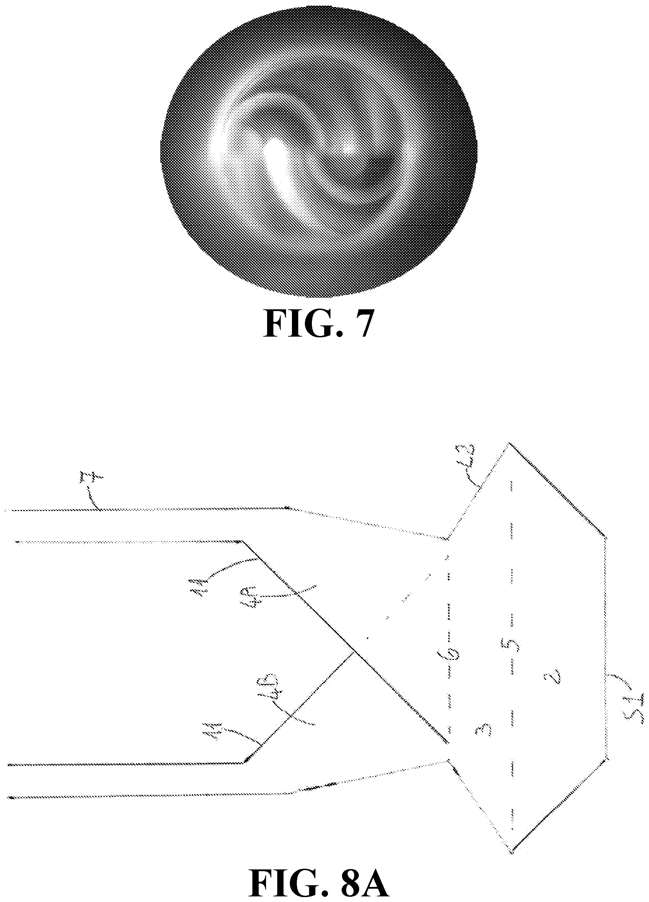

A problem with current applanation tonometer heads is the fact that intraocular pressure measurement is difficult because the light emitted by the ophthalmic dye is relatively weak. Furthermore, as shown in the picture of figure 7, the doctor who performs the measurement also sees intense light strips that disturb the vision of the hemicorones which have a lower light intensity.

Studies conducted by the applicant have shown that the reason why the light emitted by the ophthalmic dye is dim is primarily due to the shape of the end portion 2 of the head. Due to the phenomenon of refraction, the light generated by the slit lamp is deflected when it crosses the end portion 2 and impinges on the corneal applanation surface S1, propagating inside the tonometer head with an incidence angle of about 75°. Since the illumination of the applanation surface decreases as the angle of incidence increases, it will be poorly illuminated and thus the light emitted by the ophthalmic dye will be relatively low and dim.

To overcome this drawback, a rotationally symmetrical head for an applanation tonometer as defined in claim 1 has been made, having a portion delimited by a respective major base and a respective minor base and a respective external lateral surface having at least one inclined zone with respect to at the base greater than a respective internal angle between 0° and 80°. This zone is configured to define a straight path of light so that a beam of light incident on the external lateral surface from the outside of the applanation head enters the second portion and propagates in the end portion to the flat corneal applanation surface without making the light beam be reflected and without making it encounter surfaces of discontinuity of material. According to one aspect, this internal angle can be less than 75°, or 70°, or 65°, or even 60°. According to one aspect, this internal angle can be greater than 5°, or 10°, or 15°, or even 20°. Possible values for this internal angle will be 25°, or 30°, or 35°, or 40° or 45°, or 50° or 55° or values between them.

The applanation tonometer head will comprise two semi-cylindrical or semi-elliptical prisms, each having:

- a respective lower semicircular or semi-elliptical base integral with the second portion in the shape of a truncated cone and arranged along a fourth plane orthogonal to the longitudinal axial direction;
- a nominal height at a center of the respective semicircular or semi-elliptical lower base;
- a semi-elliptical upper base surface opposite the respective lower base and inclined with respect to it by a respective internal angle comprised between 10° and 60°.

The two semi-cylindrical or semi-elliptical prisms are opposite each other in the direction of the height along an axial separation plane so that the respective lower semicircular or semi-elliptical bases lie on the fourth orthogonal plane composing together a full circle or a full ellipse, respectively. The end portion, the second portion and the two prisms are integral with each other.

Other embodiments are defined in the attached claims.

Figure 1 shows a Goldmann tonometer.
Figure 2 is a perspective view of a classic applanation tonometer head.
Figure 3 shows a classic head mounted in a holding ring of an applanation tonometer, illuminated by a blue beam of light from a slit lamp.
Figure 4 shows the known head of the Goldmann tonometer applied to a patient's eye;
Figure 5 illustrates the principle of measuring intraocular pressure with the Goldmann tonometer.
Figure 6 shows semicircles or hemicorons visualized with the Goldmann tonometer, in the following cases:

a) excessive use of fluorescein; b) excessive applanation force; c) insufficient applanation force; d) hemicorones correctly aligned.

Figure 7 is a picture of the image that the physician sees when measuring the intraocular pressure of a patient with a Goldmann applanation tonometer with a known head of the type shown in Figure 2.

Figure 8A shows an axial sectional view of an applanation tonometer head with a circular or elliptical applanation surface and with a tubular shank surrounding the prisms and with a circular or elliptical applanation surface.

Figure 8B shows an axial sectional view of an applanation tonometer head with a circular or elliptical applanation surface and with a tubular shank surrounding the prisms, with a circular or elliptical applanation surface and with a cylindrical end portion.

Figure 9 shows an axial sectional view of an applanation tonometer head with a circular or elliptical applanation surface and with the prisms defined at an end longitudinally opposite the applanation surface and with a tubular shank surrounding the prisms.

Figure 10 shows an axial sectional view of an applanation tonometer head with a circular or elliptical applanation surface and with prisms defined at one end longitudinally opposite the applanation surface, with an intermediate body in the shape of two opposite truncated cones.

Figure 11 shows an axial sectional view of an applanation tonometer head with a circular or elliptical applanation surface and with prisms defined at one end longitudinally opposite the applanation surface, with an intermediate body in the shape of two opposite truncated cones and with a cylindrical end portion.

Figure 12 shows a side view of an applanation tonometer head with a circular or elliptical applanation surface and with prisms defined at one end longitudinally opposite the applanation surface, with an intermediate body shaped like three truncated cones opposite one another.

Figure 13 shows an axial sectional view of an applanation tonometer head with a circular or elliptical applanation surface and with prisms defined at one end longitudinally opposite the applanation surface, with an intermediate body shaped as three truncated cones opposite one another and with a cylindrical end portion.

Figure 14 is a perspective view of a tonometer head corresponding to the axial sectional view of Figure 8A.

Figure 15 is a perspective view of a tonometer head corresponding to the view of Figure 12.

The various innovative technical characteristics of the heads for applanation tonometers according to the present disclosure will be illustrated with the aid of the attached figures, which refer to the simplest case in which the second portion 3 of the head, which has an external lateral surface L2 with at least a zone inclined with respect to the corneal applanation surface S1 by a respective internal angle between 0° and 80°, is a truncated cone.

According to an aspect shown in Figure 8A, an applanation tonometer head 1 according to the present disclosure is made of transparent material, has rotational symmetry and comprises an end portion 2, which defines a corneal applanation surface S1 which can be for example with circular or elliptical section, a second portion 3 in the shape of a truncated cone integral with the end portion 2 and the two prisms 4a and 4b typical of known tonometer heads. The second portion 3 has a respective major base surface 5, shared with the end portion 2 and lying on a second plane S2 orthogonal to the longitudinal axial direction and having a second radius or second semi-axis, and a respective minor base surface 6 along a third transverse section S3 arranged on a third plane orthogonal to the longitudinal axial direction A and having a third radius or third semi-axis smaller than the second radius or second semi-axis, respectively. The second portion 3, which in the shown example is shaped like a second truncated cone, thus has a respective external lateral surface L2 with inclined generatrices with respect to the corneal applanation surface S1, and therefore also to the second base of the end portion 2, of a respective internal angle between 0° and 80°.

The second portion 3 is configured so that a beam of light which strikes the external lateral surface L2 and which propagates in the end portion 2, passes from the second portion 3 to the end portion 2 and impacts on the corneal applanation surface S1 without crossing surfaces of discontinuity of material and without being reflected. It will be understood that it is sufficient that there is even only one area of the external lateral surface L2 of the second portion 3 that enjoys this property so that the illumination of the corneal applanation surface S1 is enormously improved. According to one aspect, the external lateral surface L2 is oriented so that a ray of light incident perpendicularly on it enters the head and hits the corneal applanation surface S1, improving its illumination.

Studies carried out by the Applicant showed that the illumination of the corneal applanation surface S1 increases as the incidence angle of the blue light beam on the outer lateral surface L2 and on the corneal applanation surface S1 decreases. In particular, as the angle of incidence on the external lateral surface L2 decreases, the intensity of the light beam which is transmitted through the head increases; as the angle of incidence on the corneal applanation surface S1 decreases, its illumination increases. Since it is not possible to direct the blue excitation light beam perpendicular to the corneal applanation surface S1, i.e. along the longitudinal axial direction of the head, as the excitation light is generated by a slit lamp located next to the head to tonometer, according to this disclosure the tonometer head is made so that the external lateral surface L2 forms an acute internal angle with the corneal applanation surface S1, for example an internal angle that can be between 0° and 80°, or even an internal angle that can be between 5° and 60°, or an internal angle that can be between 10° and 45°. According to a preferred but not essential aspect shown in the figures, the end portion 2 has the shape of a truncated cone of which the corneal applanation surface S1 constitutes the minor base and whose major base surface coincides with the major base surface 5 of the second portion 3 in the form of a truncated cone.

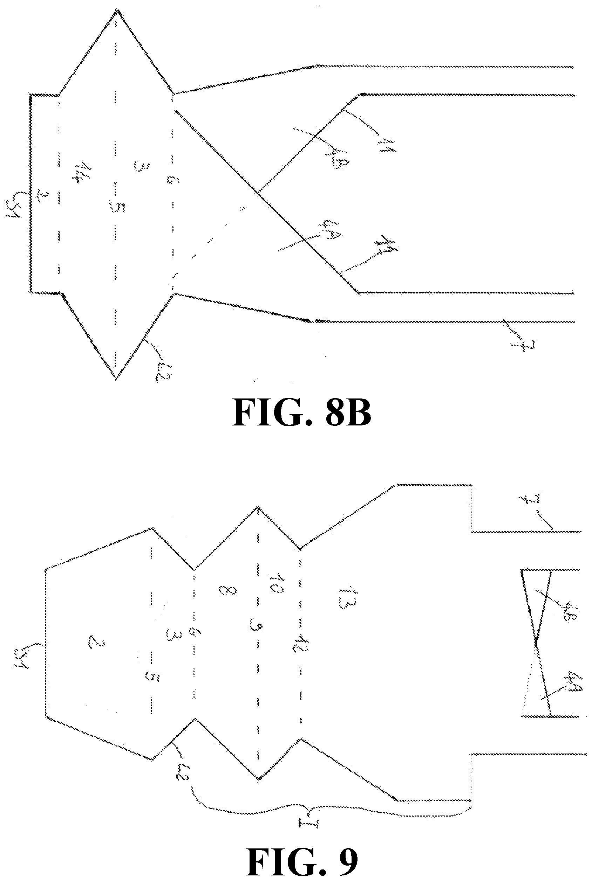

According to an aspect shown in Figures 8B, 11 and 13, the end portion 2 is shaped like a straight cylinder of which the corneal applanation surface S1 constitutes one of the two bases. It is also noted a first portion 14 in the shape of a truncated cone (but can also be in the shape of a cylinder) having a respective lower circular or elliptical base, having a first radius or first semi-axis, which is parallel to the flat corneal applanation surface S1 and it is distinct from it. This first portion 14 has a respective upper base opposite the respective lower base and is integral with the end portion 2, the second portion 3 and the two prisms 4a, 4b.

In the embodiment of the aforementioned figures, the end portion 2 has a respective shape of a straight cylinder in which a respective lower base coincides with the flat corneal applanation surface S1 and has a radius or semi-axis of applanation smaller than the first ray or first semi-axis.

According to an aspect not shown in the figures, the end portion 2 is in the shape of a truncated cone, in which the corneal applanation surface S1 constitutes the major base of the end portion 2 and the major base surface 5 of the second portion 3 forms the minor basis. According to a further aspect, in this circumstance the end portion 2 and the second portion 3 together constitute a single truncated cone with a single external lateral surface L2 without interruption. Only a relatively small central part of the corneal applanation surface S1 is used in the measurement of intraocular pressure, so the latter embodiment seems less convenient because it increases the corneal applanation surface S1 to the point that most of it produces no useful effects and helps to weigh down the head.

According to an aspect shown in the figures, the two prisms 4a and 4b are semi-cylindrical or semi-elliptical and each have:

- a respective lower semicircular or semi-elliptical base integral with the second portion 3 in the shape of a second truncated cone and arranged along a fourth plane orthogonal to the longitudinal axial direction;
- a nominal height at a center of the respective semicircular or semi-elliptical lower base;
- a semi-elliptical upper base surface 11 opposite the respective lower base and inclined with respect thereto by a respective internal angle comprised between 10 and 60°. Similarly to the prisms 4a and 4b of the known heads, for example the one shown in Figure 2, the two semi-cylindrical or semi-elliptical prisms 4a and 4b are opposite each other in the direction of the height along an axial separation plane so that the respective lower semicircular bases or semi-ellipticals lie on the fourth orthogonal plane composing together a full circle or a full ellipse, respectively. In the known heads, the prisms 4a and 4b are only semi-cylindrical and have respective semicircular lower bases.

According to an aspect of this disclosure, making the lower bases of semi-elliptical shape would allow to obviate or at least contribute to preventing another still unresolved inconvenience caused by the current tonometer heads.

When measuring the intraocular pressure with the Goldmann applanation tonometer, spurious light arcs are present that disturb the vision of the two hemicorons to be aligned, as can be better seen in the picture shown in figure 7. Studies conducted by the applicant have shown that these spurious light arcs are images of the edge of the classical head of the tonometer which are projected into the viewing area by the prisms 4a and 4b. Without being bound to any theory, it is believed that this is due to the fact that, in the classical head of figure 2, the edge of the tonometer head at the corneal applanation surface S1 is intensely illuminated by the light coming from the slit lamp because it strikes it by travelling through the air practically without being deflected. Consequently, the ophthalmic dye near the edges receives a direct excitation light and therefore emits a relatively intense light. Otherwise, due to the phenomenon of refraction at the interface between air-side surface of the end portion 2, the slit lamp light must be inclined at a relatively large angle of incidence with respect to the applanation surface in order to strike the inner part of the surface of corneal applanation S1, whereby the illumination of the corneal applanation surface S1 is poor and consequently the light emitted by the ophthalmic dye in the center of the corneal applanation surface S1 is dim.

This phenomenon can also be noted in the photograph of figure 3, in which it is seen that the blue beam emitted by the slit lamp and directed towards the applanation surface of the head illuminates a perimeter part of the head more than its center. In figure 7 it can be seen that the image of the part of the edge of the tonometer head which is located near the plane of separation of the two prisms 4a and 4b from the side of the part of the prism that is furthest away from the applanation surface, is that which more "enters" the doctor's field of vision during the measurement and constitutes a significant disturbance. To overcome this drawback, the second truncated cone-shaped portion 3 is made to have a smaller base 6 smaller than the corneal applanation surface S1, so that light rays emitted along the edge of the corneal applanation surface S1 and that propagate inside the head directed with an inclination to enter the doctor's field of vision, are intercepted by the L2 surface before reaching the prisms 4a and 4b and therefore cannot be seen by the doctor because they are shielded. To help overcome this problem further, according to an aspect of the present disclosure, the end portion 2 of the head is elliptical with the major half-shafts directed along the plane of separation of the two prisms 4a and 4b, so as to maximize the distance between the axis of the head and the portion of the perimeter edge of the applanation surface causing the disturbance.

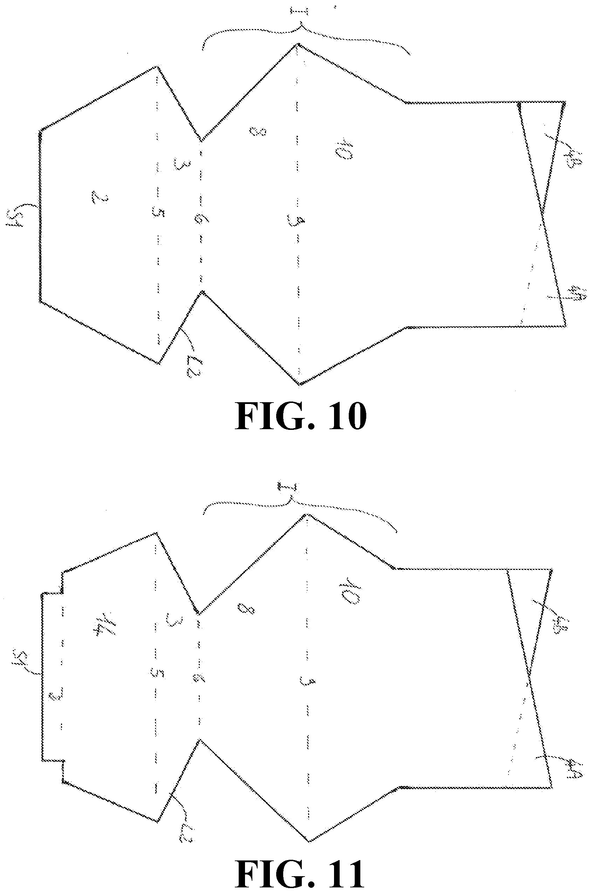

According to one aspect, shown in Figures 8A, 8B and 10, an applanation tonometer head according to this disclosure comprises a tubular shank 7 of the second portion 3 in the shape of a second truncated cone, in which the tubular shank 7 surrounds the base surfaces of the two prisms 4a and 4b and is configured to be engaged in a holding ring of an applanation tonometer while keeping the tonometer head cantilevered outside the holding ring.

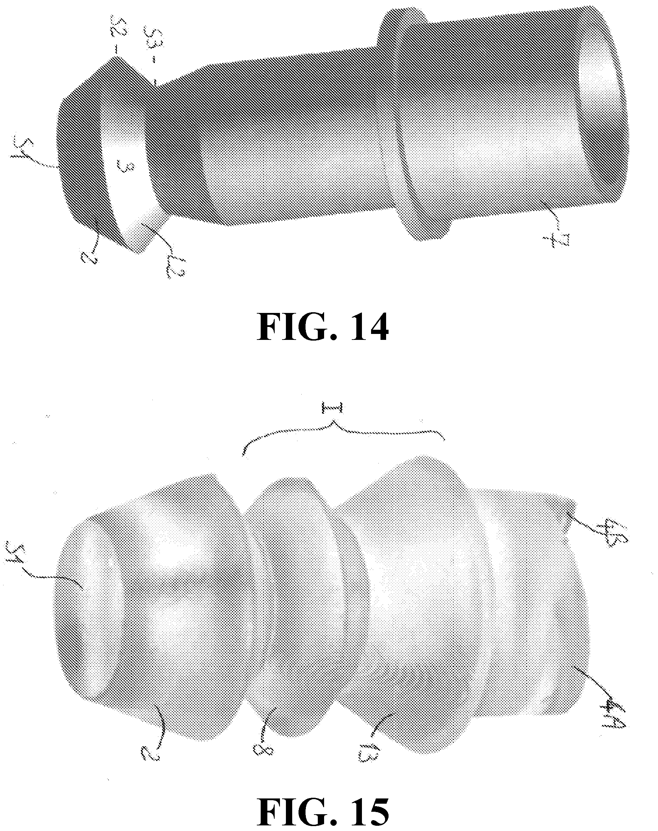

A perspective view of the head corresponding to the axial section of figure 7 is shown in figure 14. The two prisms 4a and 4b are inside and are surrounded by the tubular shank 7, which serves to engage the head in the holding ring of the tonometer to applanation. It is also possible an embodiment (not shown) of a head corresponding to that shown in figure 14 but with an elliptical corneal applanation surface S1 with the major semi-axes oriented in the separation plane of the two prisms 4a and 4b.

According to one aspect, the upper base surfaces of the two prisms 4a and 4b are more inclined with respect to the corneal applanation surface S1 the smaller the distance of these upper base surfaces from the corneal applanation surface S1.

According to one aspect, the inclination of the upper base surfaces of the two prisms 4a and 4b is determined so that their distance from the corneal applanation surface S1 is sufficiently large to allow the two prisms 4a and 4b to be inserted directly into the holding ring of an applanation tonometer, which holds the tonometer head cantilevered outside the holding ring, with the tonometer head having an approximately 2cm long portion held cantilevered outside the tonometer holding ring. For this purpose, the inclination of the base surfaces of the two prisms 4a and 4b as well as their distance from the corneal applanation surface S1 of the head will be determined in a coordinated manner so that the image of the circular annulus of ophthalmic dye that is formed during the measurement of intraocular pressure appears split - to the physician taking the measurement - into two hemicorons that "touch" as shown in figure 6D, when the portion of cornea that is flattened has a diameter of 3.06 mm.

This configuration, shown in Figures 9-13 and 15, is advantageous in that the holding ring of the applanation tonometer laterally shields the upper base surfaces of the two prisms 4a and 4b preventing interference light rays coming from the side from going to strike the respective upper base surfaces of the prisms 4a and 4b.

With this configuration, it is no longer necessary to provide an opaque protective cover on the prisms 4a and 4b on the tonometer head as an element - the holding ring of the tonometer - already present in the applanation tonometers is used. Consequently, such tonometer heads can be more easily and quickly manufactured as they do not require a second molding operation of an opaque side cover, to shield the prisms 4a and 4b from the light coming from the side. The fact of being able to use the two prisms 4a and 4b to support the cantilevered head in the holding ring of the tonometer without having to use a tubular shank 7, entails some further advantages. For example, the head shown in Figures 10 to 13 and 15 does not define internal cavities, as instead it happens in known tonometer heads, so that it can be made by milling on the lathe in a single operation by moving the tool parallel in the longitudinal axial direction of the head to define its lateral surface, without having to turn the head of the tonometer to excavate the cavity in the longitudinal axial direction. This is particularly advantageous for making glass heads. Furthermore, the upper base surfaces of the two prisms 4a and 4b occupy all the available space inside the holding ring of the tonometer, so that the head of the tonometer provides a wider image of the portion of the cornea that has been flattened. Finally, the absence of cavities allows better disinfection and cleaning of the fabricated tonometer head.

To avoid problems of total internal reflection that could lead to the formation of further spurious images in the field of vision which, emerging from the upper base surfaces of the two prisms 4a and 4b parallel to the longitudinal axial direction, would disturb the vision of the two hemicorons, the heads of the present disclosure can be made as shown in Figures 9 to 13 and 15 and comprise an intermediate body I substantially rotationally symmetrical disposed between the second portion 3 on one side and the two semi-cylindrical prisms 4a and 4b from another, along the longitudinal axial direction. The intermediate body I is integral with the second portion 3 and the two semi-cylindrical or semi-elliptical prisms 4a and 4b, and has a respective lower base surface lying in the third orthogonal plane and a respective upper base lying in the fourth plane orthogonal to the longitudinal axial direction.

This intermediate body I is shaped so that spurious light rays coming from the corneal applanation surface S1 and that do not propagate directly towards the inclined upper base surfaces of the prisms 4a and 4b, exit from the tonometer head or are internally reflected. If these spurious light rays were able to impinge on the inclined surfaces of the prisms 4a and 4b, they would do so with angles of incidence such as not to emerge along directions parallel to the longitudinal axial direction of the tonometer head. In this way, they are not visible to the operator, since the observation point of the operator is relatively distant from the head of the tonometer and is substantially oriented according to the longitudinal axial direction of the head.

According to one aspect, shown in Figures 10 and 11, the intermediate body I is composed of:

- a third truncated cone 8 having a respective minor base surface 6 constituting the lower base surface 6 of the intermediate body I and coinciding with the minor base surface 6 of the second truncated cone-shaped portion 3, and having a respective major base surface 9 arranged along a fifth plane orthogonal to the longitudinal axial direction;
- a fourth truncated cone 10 having a respective major base surface 9 coincident with the major base surface 9 of the third truncated cone 8, and having a respective minor base surface constituting the upper base surface of the intermediate body I and arranged along the fourth plane orthogonal to the longitudinal axial direction.

In this configuration, the holding ring of the tonometer will abut against the lateral surface of the fourth truncated cone 10, blocking the head with the prisms 4a and 4b inserted in the holding ring.

According to one aspect, notches are made on the external lateral surface L2 of the third truncated cone 8 or of the fourth truncated cone 10 (or both), for example by screen printing or engraving or even gluing, so as to allow the operator to arrange the head of the tonometer by correctly orienting the plane of separation of the two prisms 4a and 4b in space.

According to one aspect, shown in Figures 9, 12, 13 and 15, the intermediate body I comprises:

- a third truncated cone 8 having a respective minor base surface 6 constituting the lower base surface 6 of the intermediate body I and coinciding with the minor base surface 6 of the second truncated cone-shaped portion 3, and having a respective major base surface 9 arranged along a fifth plane orthogonal to the longitudinal axial direction;
- a fourth truncated cone 10 having a respective major base surface 9 coincident with the major base surface 9 of the third truncated cone 8, and having a respective minor base surface 12 arranged along a sixth plane orthogonal to the longitudinal axial direction;
- a fifth truncated cone 13 having a respective minor base surface 12 coinciding with the minor base surface 12 of the fourth truncated cone 10, and having a respective major base surface constituting the upper base surface of the intermediate body I and arranged along the fourth plane orthogonal to the longitudinal axial direction.

In this configuration, the tonometer head has an upper surface, given by the larger base surface of the fifth truncated cone 13, which abuts against the holding ring of the tonometer, as in classical tonometer heads. As can be easily understood, the second portion 3 and the third truncated cone 8 can be made, instead of having a conical shape, so as to form together a hyperboloid of one sheet. Similarly, also the fourth truncated cone 10 and the fifth truncated cone 13 can be made so as to form together a respective hyperboloid of one sheet. Alternatively, each of the truncated cones shown in the figures could be replaced with a truncated solid having a lateral surface which corresponds to a respective hyperboloid of two sheets.

According to an aspect (not shown in the figures) of the head for applanation tonometer of this disclosure, the end portion 2 and the second portion 3 are truncated cones with lateral surfaces aligned with coincident generatrices so as to constitute a single truncated cone with a single side surface without solution of continuity.

According to one aspect of the head for an applanation tonometer of this disclosure, the end portion 2 and the second portion 3 have respective elliptical bases having respective major semi-axes lying in the separation plane of the two prisms 4a and 4b.

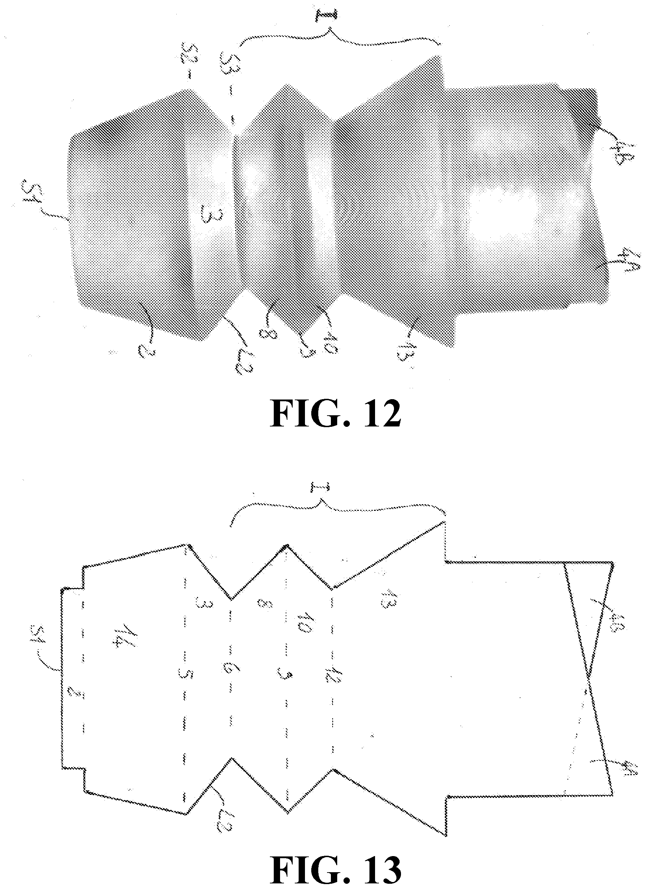

A perspective view of the head corresponding to the view of figure 12 is shown in figure 15. The two prisms 4a and 4b which constitute the end of the head longitudinally opposite to the corneal applanation surface S1 are shown, configured so that they can be inserted into the holding ring of the applanation tonometer to keep the head cantilevered out of it. Conveniently, the inclination of the upper base surface 11 of the prisms 4a and 4b will be dimensioned so that the height of the prisms 4a and 4b is such that the upper bases remain inside the holding ring and do not protrude from it, thus being shielded laterally from disturbing light rays.

A head corresponding to the one shown in figure 15 could also be made but with an elliptical corneal applanation surface S1 (not shown) with the major semi-axes oriented in the separation plane of the two prisms 4a and 4b. This embodiment appears less convenient because, since it is no longer a piece having an externally circular symmetry around the longitudinal axial direction, it is more difficult to manufacture on the lathe. According to one aspect, on the external lateral surface L2 of the third truncated cone 8 or of the fourth truncated cone 10 or of the fifth truncated cone 13 (or of all or part of them or of the lateral surfaces of the single-pitched hyperboloids) notches, for example by screen printing or engraving or even gluing, so as to allow the operator to arrange the head of the tonometer by correctly orienting the plane of separation of the two prisms 4a and 4b in space.

According to one aspect, the intermediate body I has opaque external lateral surfaces so as to shield rays of light which from the outside should be directed towards the inside of the tonometer head. However, this aspect does not appear indispensable as light rays that hit the tonometer head laterally, even if they enter the intermediate body I, are unable to propagate up to the inclined surfaces of the two prisms 4a and 4b to emerge from them in a direction parallel to the direction longitudinal axial of the head, therefore they do not disturb the doctor during the measurement. The fact that it is not necessary to shield the lateral surface of the intermediate body I means that it is possible to avoid the step of molding the side cover, in rubber or low melting plastic, around the head of the tonometer. This operation is still necessary for current plastic tonometer heads and is particularly critical as it is carried out with a heat treatment performed downstream of the injection molding of the tonometer head. For this reason, in current heads made of PMMA or PMA this second molding operation, even if performed at a lower temperature, is likely to alter the perfectly flat shape of the inclined surfaces of the prisms 4a and 4b, distorting the image seen by the doctor.

According to one aspect, the third truncated cone 8 has a respective external lateral surface having generatrices whose extension passes through internal points of said flat circular applanation surface S1. This feature prevents light rays coming from the edges of the corneal applanation surface S1 from being deflected towards the upper base surfaces of the two prisms 4a and 4b.

According to one aspect, not shown in the drawings, the generatrices of the second truncated cone-shaped portion 3 are not straight but curved, oriented so as to direct their concavity towards the corneal applanation surface S1. In this case, the generatrices will have points with an inclination with respect to the corneal applanation surface S1 between 0° and 80°, so that the external lateral surface L2 of the second portion 3 will be convex and in any case will be at least locally oriented so as to allow a better illumination of the corneal applanation surface S1, as in the heads shown in figures 7 to 12. However, this aspect is less preferable since a rounded external lateral surface L2 for the second portion 3 would cause focusing phenomena of the blue light beam excitation, which could disturb the patient during the measurement.

**Claims**

1. An applanation tonometer head, comprising a transparent integral body with rotational symmetry having a longitudinal axial direction, defining:

   at a first end along said longitudinal axial direction, an end portion (2) defining a flat circular or elliptical corneal applanation surface (S1), having a respective applanation radius or semi-axis, arranged on a first base plane of the end portion (2) orthogonal to a longitudinal axis of the tonometer head, and having a second base parallel to said flat corneal applanation surface (S1); a second portion (3), distinct from said end portion (2) and integral with it, having a respective major base having a second radius or second semi-axis and arranged on a second plane (S2) orthogonal to said longitudinal axial direction, and a respective minor base along a third cross section (S3) arranged on a third plane orthogonal to said longitudinal axial direction and having a third radius or third semi-axis smaller than said second radius or second semi-axis, respectively, said second portion (3) having a respective external lateral surface (L2) comprised between said major base and said minor base, at least a zone of said external lateral surface (L2) being inclined with respect to said corneal applanation surface (S1) by a respective internal angle comprised between 0° and 80° and being configured to define a rectilinear light path so that a light shaft incident on the external lateral surface (L2) from outside of the applanation head enters said second portion (3) and propagates in said end portion (2) up to said flat corneal applanation surface (S1) without to be reflected and without encountering material discontinuity surfaces; two semi-cylindrical or semi-elliptical prisms (4a, 4b), each having:

   - a respective semicircular or semi-elliptical lower base integral with said second portion (3) and arranged along a fourth plane orthogonal to said longitudinal axial direction;
   - a nominal height at a center of the respective semi-circular or semi-elliptical lower base;
   - a semi-elliptical upper base surface (11) opposite to said respective lower base and inclined with respect to the lower base by a respective internal angle comprised between 10° and 60°;

   said two semi-cylindrical or semi-elliptic prisms (4a, 4b) being opposed in the sense of height along an axial separation plane so that the respective semicircular or semi-elliptic lower bases lie on said orthogonal fourth plane composing together a full circle or a full ellipse, respectively; wherein said end portion (2), said second portion (3) and said two prisms (4a, 4b) are integral with each other.

2. The applanation tonometer head according to claim 1, wherein said second portion (3) is in the shape of a truncated cone and the external lateral surface (L2) is defined by directices inclined with respect to said corneal applanation surface (S1) by a respective internal angle comprised between 0° and 80°.

3. The applanation tonometer head according to claim 1 or 2, wherein said fourth orthogonal plane coincides with said third orthogonal plane.

4. The applanation tonometer head according to claim 1 or 2, comprising an intermediate body (I) with rotational symmetry arranged between said second portion (3) on one side and said two semi-cylindrical prisms (4a, 4b) on the other side other along said longitudinal axial direction , said intermediate body (I) being integral with said second portion (3) and with said two semi-cylindrical prisms (4a, 4b), said intermediate body (I) having a respective lower base surface lying in said third orthogonal plane and a respective upper base lying in said fourth orthogonal plane.

5. The applanation tonometer head according to claim 4, wherein said intermediate body (I) is composed of:

a third truncated cone (8) having a respective minor base surface (6) constituting the lower base surface of the intermediate body (I) and coinciding with the minor base surface (6) of said second portion (3), and having a respective larger base surface (9) arranged along a fifth plane orthogonal to said longitudinal axial direction;
a fourth truncated cone (10) having a respective major base surface (9) coinciding with the major base surface of said third truncated cone (8), and having a respective minor base surface (6) constituting the base upper surface (11) of the intermediate body (I) and arranged along said fourth plane orthogonal to said longitudinal axial direction.

6. The applanation tonometer head according to claim 4, wherein said intermediate body (I) is composed of:

a third truncated cone (8) having a respective minor base surface (6) constituting the lower base surface of the intermediate body (I) and coinciding with the minor base surface (6) of said second portion (3), and having a respective major base surface (9) arranged along a fifth plane orthogonal to said longitudinal axial direction ;
a fourth truncated cone (10) having a respective major base surface (9) coinciding with the major base surface of said third truncated cone (8), and having a respective minor base surface (6) arranged along a sixth plane orthogonal to said longitudinal axial direction;
a fifth truncated cone (13) having a respective minor base surface (6) coinciding with the minor base surface (6) of said fourth truncated cone (10), and having a respective major base surface (9) constituting the upper base surface (11) of the intermediate body (I) is arranged along said fourth plane orthogonal to said longitudinal axial direction.

7. The applanation tonometer head according to one of claims 1 to 6, wherein said third beam or third semi-axis is smaller than said applanation radius or semi-axis.

8. The applanation tonometer head according to claim 5, wherein said third truncated cone (8) has a respective external lateral surface (L2) having generators whose extension passes through internal points of said flat circular or elliptical corneal applanation surface (S1).

9. The applanation tonometer head according to one of claims 1 to 8, comprising a tubular tang (7) of said second portion (3) in the shape of a truncated cone, in which said tubular tang (7) surrounds the upper base surfaces of the two prisms (4a, 4b) and is configured to be inserted in a holding ring of an applanation tonometer maintaining cantilevered the tonometer head outside of the holding ring.

10. The applanation tonometer head according to one of claims 1 to 8, wherein said two prisms (4a, 4b) are configured to be inserted into a holding ring of an applanation tonometer and in direct contact with it, maintaining cantilevered the tonometer head outside the holding ring.

11. The applanation tonometer head according to one of claims 1 to 10, comprising:

a cylinder-shaped or truncated cone-shaped first portion having a respective circular or elliptical lower base having a first radius or first semi-axis, said lower base is parallel to and is distinct from said flat corneal applanation surface (S1), said first portion having a respective upper base opposite to said respective lower base;
wherein said end portion (2) has a respective straight cylinder shape in which a respective lower base coincides with said flat corneal applanation surface (S1) and has an applanation radius or semi-axis smaller than the first radius or first semi-axis, respectively;
said end portion (2), said first portion, said second portion (3) and said two semi-cylindrical prisms (4a, 4b) are integral with each other.

**Patentansprüche**

1. Applanationstonometerkopf, umfassend einen transparenten Integralkörper mit Rotationssymmetrie, der eine axiale Längsrichtung aufweist, der definiert:

an einem ersten Ende entlang der axialen Längsrichtung einen Endabschnitt (2), der eine flache kreisförmige oder elliptische Hornhautapplanationsoberfläche (S1) definiert, die einen jeweiligen Applanationsradius oder eine Halbachse aufweist, der/die auf einer ersten Basisebene des Endabschnitts (2) orthogonal zu einer Längsachse des Tonometerkopfes angeordnet ist, und eine zweite Basis parallel zu der flachen Hornhautapplanationsoberfläche (S1) aufweist; einen zweiten Abschnitt (3), der sich von dem Endabschnitt (2) unterscheidet und mit diesem integral ist, der eine jeweilige Hauptbasis aufweist, die einen zweiten Radius oder eine zweite Halbachse aufweist und auf einer zweiten Ebene (S2) orthogonal zu der axialen Längsrichtung angeordnet ist, und eine jeweilige Nebenbasis entlang eines dritten Querschnitts (S3), die auf einer dritten Ebene orthogonal zu der axialen Längsrichtung angeordnet ist und einen dritten Radius oder eine dritte Halbachse aufweist, der/die kleiner als der zweite Radius beziehungsweise die zweite Halbachse ist, wobei der zweite Abschnitt (3) eine jeweilige äußere Seitenoberfläche (L2) aufweist, die zwischen der Hauptbasis und der Nebenbasis enthalten ist, wobei mindestens eine Zone der äußeren Seitenoberfläche (L2) hinsichtlich der Applanationsoberfläche (S1) um einen jeweiligen inneren Winkel geneigt ist, der zwischen 0° und 80° enthält und konfiguriert ist, um einen geradlinigen Lichtpfad zu definieren, sodass ein Lichtstrahleinfall auf der äußeren Seitenoberfläche (L2) von außerhalb des Applanationskopfes in den zweiten Abschnitt (3) eintritt und sich in den Endabschnitt (2) bis zu der Hornhautapplanationsoberfläche (S1) ausbreitet, ohne reflektiert zu werden und ohne auf Materialunterbrechungsoberflächen zu treffen; zwei halbzylindrische oder halbelliptische Prismen (4a, 4b), die jeweils aufweisen:

- ein jeweiliges halbkreisförmiges oder halbelliptisches untere Basisintegral, das mit dem zweiten Abschnitt (3) integral ist und entlang einer vierten Ebene orthogonal zu der axialen Längsrichtung angeordnet ist;
- eine nominelle Höhe in einer Mitte der jeweiligen halbkreisförmigen oder halbelliptischen unteren Basis;
- eine halbelliptische obere Basisoberfläche (11), die der jeweiligen unteren Basis gegenüberliegt und hinsichtlich der unteren Basis um einen jeweiligen inneren Winkel geneigt ist, der zwischen 10° und 60° enthält;

wobei die zwei halbzylindrischen oder halbelliptischen Prismen (4a, 4b) im Sinne der Höhe entlang einer axialen Trennungsebene entgegengesetzt sind, sodass die jeweiligen halbkreisförmigen oder halbelliptischen unteren Basen auf der orthogonalen vierten Ebene liegen, die zusammen einen vollen Kreis oder eine volle Ellipse ausbilden; wobei der Endabschnitt (2), der zweite Abschnitt (3) und die zwei Prismen (4a, 4b) miteinander integral sind.

2. Applanationstonometerkopf nach Anspruch 1, wobei der zweite Abschnitt (3) in der Form eines Kegelstumpfes ist und die äußere Seitenoberfläche (L2) durch Richtungen definiert ist, die hinsichtlich der Hornhautapplanationsoberfläche (S1) um einen jeweiligen inneren Winkel geneigt ist, der zwischen 0° und 80° enthält.

3. Applanationstonometerkopf nach Anspruch 1 oder 2, wobei die vierte orthogonale Ebene mit der dritten orthogonalen Ebene zusammenfällt.

4. Applanationstonometerkopf nach Anspruch 1 oder 2, umfassend einen Zwischenkörper (I) mit Rotationssymmetrie, die zwischen dem zweiten Abschnitt (3) auf einer Seite und den zwei halbzylindrischen Prismen (4a, 4b) auf der anderen Seite entlang der axialen Längsrichtung angeordnet ist, wobei der Zwischenkörper (I) mit dem zweiten Abschnitt (3) und mit den zwei halbzylindrischen Prismen (4a, 4b) integral ist, wobei der Zwischenkörper (I) eine jeweilige untere Basisoberfläche aufweist, die in der dritten orthogonalen Ebene liegt, und eine jeweilige obere Basis, die in der vierten orthogonalen Ebene liegt.

5. Applanationstonometerkopf nach Anspruch 4, wobei der Zwischenkörper (I) ausgebildet ist aus:

einem dritten Kegelstumpf (8), der eine jeweilige Nebenbasisoberfläche (6) aufweist, die die untere Basisoberfläche des Zwischenkörpers (I) bildet und mit der Nebenbasisoberfläche (6) des zweiten Abschnitts (3) zusammenfällt und der eine jeweilige größere Basisoberfläche (9) aufweist, die entlang einer fünften Ebene orthogonal zu der axialen Längsrichtung angeordnet ist; einem vierten Kegelstumpf (10), der eine jeweilige Hauptbasisoberfläche (9) aufweist, die mit der Hauptbasisoberfläche des dritten Kegelstumpfes (8) zusammenfällt, und der eine jeweilige Nebenbasisoberfläche (6) aufweist, die die obere Basisoberfläche (11) des Zwischenkörpers (I) bildet und entlang der vierten Ebene orthogonal zu der axialen Längsrichtung angeordnet ist.

6. Applanationstonometerkopf nach Anspruch 4, wobei der Zwischenkörper (I) ausgebildet ist aus:

einem dritten Kegelstumpf (8), der eine jeweilige Nebenbasisoberfläche (6) aufweist, die die untere Basisoberfläche des Zwischenkörpers (I) bildet und mit der Nebenbasisoberfläche (6) des zweiten Abschnitts (3) zusammenfällt und der eine jeweilige Hauptbasisoberfläche (9) aufweist, die entlang einer fünften Ebene orthogonal zu der axialen Längsrichtung angeordnet ist; einem vierten Kegelstumpf (10), der eine jeweilige Hauptbasisoberfläche (9) aufweist, die mit der Hauptbasisoberfläche des dritten Kegelstumpfes (8) zusammenfällt, und der eine jeweilige Nebenbasisoberfläche (6) aufweist, die entlang einer sechsten Ebene orthogonal zu der axialen Längsrichtung angeordnet ist; einem fünften Kegelstumpf (13), der eine jeweilige Nebenbasisoberfläche (6) aufweist, die mit der Nebenbasisoberfläche (6) des vierten Kegelstumpfes (10) zusammenfällt, und der eine jeweilige Hauptbasisoberfläche (9) aufweist, die die obere Basisoberfläche (11) des Zwischenkörpers (I) bildet, entlang der vierten Ebene orthogonal zu der axialen Längsrichtung angeordnet ist.

7. Applanationstonometerkopf nach einem der Ansprüche 1 bis 6, wobei der dritte Strahl oder die dritte Halbachse kleiner als der Applanationsradius oder die Halbachse ist.

8. Applanationstonometerkopf nach Anspruch 5, wobei der dritte Kegelstumpf (8) eine jeweilige äußere Seitenoberfläche (L2) aufweist, die Generatoren aufweist, deren Verlängerung durch innere Punkte der flachen kreisförmigen oder elliptischen Hornhautapplanationsoberfläche (S1) verläuft.

9. Applanationstonometerkopf nach einem der Ansprüche 1 bis 8, umfassend ein rohrförmiges angespitztes Ende (7) des zweiten Abschnitts (3) in der Form eines Kegelstumpfes, in dem das rohrförmige angespitzte Ende (7) die oberen Basisoberflächen der zwei Prismen (4a, 4b) umgibt und konfiguriert ist, um in einen Haltering eines Applanationstonometers eingeführt zu werden, der den Tonometerkopf außerhalb des Halterings freitragend hält.

10. Applanationstonometerkopf nach einem der Ansprüche 1 bis 8, wobei die zwei Prismen (4a, 4b) konfiguriert sind, um in einen Haltering eines Applanationstonometers eingeführt zu werden und in direktem Kontakt mit diesem zu stehen, wobei der Tonometerkopf außerhalb des Halterings freitragend gehalten wird.

11. Applanationstonometerkopf nach einem der Ansprüche 1 bis 10, umfassend:

einen zylindrischen oder kegelstumpfförmigen ersten Abschnitt, der eine jeweilige kreisförmige oder elliptische untere Basis aufweist, die einen ersten Radius oder eine erste Halbachse aufweist, wobei die untere Basis parallel zu der flachen Hornhauptapplanationsoberfläche (S1) ist und sich von dieser unterscheidet; wobei der erste Abschnitt eine jeweilige obere Basis gegenüber der jeweiligen unteren Basis aufweist; wobei der Endabschnitt (2) eine jeweilige gerade Zylinderform aufweist, in der eine jeweilige untere Basis mit der flachen Hornhautapplanationsoberfläche (S1) zusammenfällt und einen Applanationsradius oder eine Halbachse aufweist, der/die kleiner als der erste Radius beziehungsweise die erste Halbachse ist; der Endabschnitt (2), der erste Abschnitt, der zweite Abschnitt (3) und die zwei halbzylindrischen Prismen (4a, 4b) miteinander integral sind.

## Revendications

1. Tête de tonomètre à aplanation, comprenant un corps intégral transparent avec une symétrie de rotation ayant une direction axiale longitudinale, définissant :

au niveau d'une première extrémité le long de ladite direction axiale longitudinale, une partie terminale (2) définissant une surface d'aplanation cornéenne plate circulaire ou elliptique (S1), ayant un rayon ou un demi-axe d'aplanation respectif, disposée sur un premier plan de base de la partie terminale (2) orthogonal à un axe longitudinal de la tête du tonomètre, et ayant une seconde base parallèle à ladite surface d'aplanation cornéenne plate (S1) ; une seconde partie (3), distincte de ladite partie terminale (2) et solidaire de celle-ci, ayant une base principale respective ayant un deuxième rayon ou un deuxième demi-axe et disposée sur un deuxième plan (S2) orthogonal à ladite direction axiale longitudinale et une base secondaire respective le long d'une troisième section transversale (S3) disposée sur un troisième plan orthogonal à ladite direction axiale longitudinale et ayant un troisième rayon ou un troisième demi-axe plus petit que ledit deuxième rayon ou deuxième demi-axe, respectivement, ladite seconde partie (3) ayant une surface latérale externe respective (L2) comprise entre ladite base principale et ladite base secondaire, au moins une zone de ladite surface latérale externe (L2) étant inclinée par rapport à ladite surface d'aplanation cornéenne (S1) d'un angle interne respectif compris entre 0° et 80° et étant conçue pour définir un trajet lumineux rectiligne de sorte qu'un puits de lumière incident sur la surface latérale externe (L2) depuis l'extérieur de la tête d'aplanation pénètre dans ladite seconde partie (3) et se propage dans ladite partie terminale (2) jusqu'à ladite surface d'aplanation cornéenne plate (S1) sans être reflété et sans rencontrer de surfaces de discontinuité matérielle ;

deux prismes semi-cylindriques ou semi-elliptiques (4a, 4b), chacun ayant :

- une base inférieure respective semi-circulaire ou semi-elliptique solidaire avec ladite seconde partie (3) et disposée le long d'un quatrième plan orthogonal à ladite direction axiale longitudinale ;
- une hauteur nominale au niveau d'un centre de la base inférieure respective semi-circulaire ou semi-elliptique ;
- une surface de base supérieure semi-elliptique (11) opposée à ladite base inférieure respective et inclinée par rapport à la base inférieure d'un angle interne respectif compris entre 10° et 60°;

lesdits deux prismes semi-cylindriques ou semi-elliptiques (4a, 4b) étant opposés dans le sens de la hauteur le long d'un plan de séparation axial, de sorte que les bases inférieures respectives semi-circulaires ou semi-elliptiques se trouvent sur ledit quatrième plan orthogonal, composant ensemble un cercle complet ou une ellipse complète, respectivement ;

dans laquelle ladite partie terminale (2), ladite seconde partie (3) et lesdits deux prismes (4a, 4b) sont solidaires les uns des autres.

2. Tête de tonomètre à aplanation selon la revendication 1, dans laquelle ladite seconde partie (3) est en forme d'un cône tronqué et la surface latérale externe (L2) est définie par des lignes directrices inclinées par rapport à ladite surface d'aplanation cornéenne (S1) d'un angle interne respectif compris entre 0° et 80°.

3. Tête de tonomètre à aplanation selon la revendication 1 ou 2, dans laquelle ledit quatrième plan orthogonal coïncide avec ledit troisième plan orthogonal.

4. Tête de tonomètre à aplanation selon la revendication 1 ou 2, comprenant un corps intermédiaire (I) à symétrie de rotation disposé entre ladite seconde partie (3) d'un côté et lesdits deux prismes semi-cylindriques (4a, 4b) de l'autre côté le long de ladite direction axiale longitudinale, ledit corps intermédiaire (I) étant solidaire avec ladite seconde partie (3) et lesdits deux prismes semi-cylindriques (4a, 4b), ledit corps intermédiaire (I) ayant une surface de base inférieure respective située dans ledit troisième plan orthogonal et une base supérieure respective située dans ledit quatrième plan orthogonal.

5. Tête de tonomètre à aplanation selon la revendication 4, dans laquelle ledit corps intermédiaire (I) est composé de :

un troisième cône tronqué (8) ayant une surface de base secondaire respective (6) constituant la surface de base inférieure du corps intermédiaire (I) et coïncidant avec la surface de base secondaire (6) de ladite seconde partie (3), et ayant une grande surface de base respective (9) disposée le long d'un cinquième plan orthogonal à ladite direction axiale longitudinale ;
un quatrième cône tronqué (10) ayant une surface de base principale respective (9) coïncidant avec la surface de base principale dudit troisième cône tronqué (8), et ayant une surface de base secondaire respective (6) constituant la surface supérieure de base (11) du corps intermédiaire (I) et disposée le long dudit quatrième plan orthogonal à ladite direction axiale longitudinale.

6. Tête de tonomètre à aplanation selon la revendication 4, dans laquelle ledit corps intermédiaire (I) est composé de :

un troisième cône tronqué (8) ayant une surface de base secondaire respective (6) constituant la surface de base inférieure du corps intermédiaire (I) et coïncidant avec la surface de base secondaire (6) de ladite seconde partie (3) et ayant une surface de base principale respective (9) disposée le long d'un cinquième plan orthogonal à ladite direction axiale longitudinale ;
un quatrième cône tronqué (10) ayant une surface de base principale respective (9) coïncidant avec la surface de base principale dudit troisième cône tronqué (8) et ayant une surface de base secondaire respective (6) disposée le long d'un sixième plan orthogonal à ladite direction axiale longitudinale ;
un cinquième cône tronqué (13) ayant une surface de base secondaire respective (6) coïncidant avec la surface de base secondaire (6) dudit quatrième cône tronqué (10) et ayant une surface de base principale respective (9) constituant la surface de base supérieure (11) du corps intermédiaire (I) est disposé le long dudit quatrième plan orthogonal à ladite direction axiale longitudinale.

7. Tête de tonomètre à aplanation selon l'une des revendications 1 à 6, dans laquelle ledit troisième faisceau ou le troisième demi-axe est plus petit que ledit rayon ou demi-axe d'aplanation.

8. Tête de tonomètre à aplanation selon la revendication 5, dans laquelle ledit troisième cône tronqué (8) a une surface latérale externe respective (L2) ayant des générateurs dont l'extension passe par des points internes de ladite surface d'aplanation cornéenne circulaire ou elliptique plate (S1).

9. Tête de tonomètre à aplanation selon l'une des revendications 1 à 8, comprenant un tenon tubulaire (7) de ladite seconde partie (3) en forme de cône tronqué, dans laquelle ledit tenon tubulaire (7) entoure les surfaces de base supérieures des deux prismes (4a, 4b) et est conçu pour être inséré dans un anneau de maintien d'un tonomètre à aplanation maintenant en porte-à-faux la tête de tonomètre à l'extérieur de l'anneau de maintien.

10. Tête de tonomètre à aplanation selon l'une des revendications 1 à 8, dans laquelle lesdits deux prismes (4a, 4b) sont conçus pour être insérés dans un anneau de maintien d'un tonomètre à aplanation et en contact direct avec celui-ci, maintenant la tête de tonomètre en porte-à-faux à l'extérieur de l'anneau de maintien.

11. Tête de tonomètre à aplanation selon l'une des revendications 1 à 10, comprenant :

    une première partie en forme de cylindre ou de cône tronqué ayant une base inférieure respective circulaire ou elliptique ayant un premier rayon ou un premier demi-axe, ladite base inférieure étant parallèle à et distincte de ladite surface d'aplanation cornéenne plate (S1), ladite première partie ayant une base supérieure respective opposée à ladite base inférieure respective ;
    dans laquelle ladite partie terminale (2) a une forme de cylindre droit respective dans laquelle une base inférieure respective coïncide avec ladite surface d'aplanation cornéenne plate (S1) et a un rayon ou un demi-axe d'aplanation plus petit que le premier rayon ou le premier demi-axe, respectivement ;
    ladite partie terminale (2), ladite première partie, ladite seconde partie (3) et lesdits deux prismes semi-cylindriques (4a, 4b) sont solidaires les uns des autres.

EP 4 132 345 B1

FIG. 1

FIG. 2

14

**FIG. 3**

**FIG. 4**

FIG. 5

FIG. 6

**FIG. 7**

**FIG. 8A**

**FIG. 8B**

**FIG. 9**

FIG. 10

FIG. 11

**FIG. 12**

**FIG. 13**

**FIG. 14**

**FIG. 15**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6083161 A
- US 2001051770 A1